# EUROPEAN PATENT SPECIFICATION

(11) **EP 3 238 755 B1**
(45) Date of publication and mention of the grant of the patent: **20.02.2019**
(21) Application number: 15873329.5
(22) Date of filing: 25.12.2015
(51) Int. Cl.: A61M 1/16, B01F 5/06, B01F 13/10, B01F 15/00, B01F 3/08, B01F 3/22

(54) **SOLUTION PRODUCING DEVICE AND BLOOD PURIFICATION SYSTEM**
VORRICHTUNG ZUR HERSTELLUNG EINER LÖSUNG UND BLUTREINIGUNGSSYSTEM
DISPOSITIF DE PRODUCTION DE SOLUTION ET SYSTÈME DE PURIFICATION DU SANG

(30) Priority: 25.12.2014 JP 2014262916
(43) Date of publication of application: 01.11.2017
(73) Proprietor: Asahi Kasei Medical Co., Ltd., Tokyo 100-0006 (JP)
(72) Inventor: KOUDA, Masaaki, Tokyo 101-8101 (JP)
(74) Representative: dompatent von Kreisler Selting Werner - Partnerschaft von Patent- und Rechtsanwälten mbB
(86) International application number: PCT/JP2015/086360
(87) International publication number: WO 2016/104761

(56) References cited:
- JP-A- H04 200 559
- JP-B2- 4 458 346
- US-A- 4 814 073
- US-A1- 2002 012 619
- US-A1- 2004 243 094
- US-A1- 2009 084 719
- US-A1- 2009 084 721

## Description

### Technical Field

The present invention relates to a solution generating device and a blood purification system.

### Background Art

An example of a therapeutic method for severely ill patients with, for example, acute renal failure, etc. includes continuous hemofiltration therapy which continuously purifies blood through extracorporeal circulation. In the continuous hemofiltration therapy, purification treatment of blood is performed continuously for a long time in a blood purification device and thus it is preferable for dialysate and fluid replacement (hereinafter referred simply as a "solution") needed for this blood purification treatment to be able to be refilled as appropriate into the blood purification device. As a method for implementing such refilling, for example, the following steps are proposed: connecting a dialysate preparation device to a blood purification device and preparing a dialysate using said dialysate preparation device so as to supply the dialysate to the blood purification device (see patent document 1).

For example, the dialysate may be comprised of two types of undiluted solutions and water, and in the above-mentioned dialysate preparation device, the two types of undiluted solutions and water are mixed in the circuit to generate a dialysate, said generated dialysate is stored in a reservoir and supplied from the reservoir to the blood purification device.

### Citation List

### Patent Document

Patent Document 1: JP4458346B
US2009/0084719 discloses a dialysis fluid cassette including an air separation chamber.

### Summary

### Technical Problem

Since dialysate and fluid replacement used for blood purification treatment affect the body fluid amount and body fluid component of the patient, it needs to be produced so as to be at a correct concentration.

In order to control the generated solution so as to be at a correct concentration, for example, each of the two types of undiluted solutions and water may be sent by a fixed quantity using a metering piston pump so as to be mixed together. However, as metering piston pumps are costly, they cannot be disposable and thus become a burden for healthcare professionals as they will require cleaning and sterilization of circuits and pumps each time a blood purification treatment is performed.

When attempting to control the generated solution so as to be at a correct concentration using a relatively inexpensive pump, such as an ordinary tube pump, instead of using the metering piston pump, it is necessary to measure the concentration of the mixture by a sensor in the circuit and adjust the flow rate of the respective pumps for the undiluted solutions and water based on the measured concentration. However, in such a case, since the concentration is measured by the sensor during the mixing of the undiluted solutions and water in the circuit, if the undiluted solutions and water are not sufficiently mixed, the concentration cannot be correctly measured. Further, there may be cases in which the concentration may not be measured correctly by the sensor if there are bubbles within the flowing mixture. As a result, there is a possibility that the generated solution may not be controlled to the correct concentration.

The present invention has been made by taking the above points into consideration, and the present invention is intended to provide a solution generating device and a blood purification system which are disposable, able to correctly measure the concentration of the mixture, and able to correctly control the concentration of the generated solution.

### Solution to Problem

As a result of dedicated research by the inventors to achieve the above object, the inventors have found that the measurement of concentration by the sensor may be performed correctly by providing a gas discharge section in a line for mixing fluid, and thereby the flow rate of the pump may be correctly adjusted and the concentration of the generated solution may be strictly controlled. Hence the present invention has been achieved. The invention is defined in appended independent claim 1, preferred embodiments are described in the dependent claims.

### Advantageous Effects of Invention

Since the measurement of the concentration of a mixture is able to be performed correctly, and the concentration of the generated solution may be strictly controlled by the present invention, a solution with a correct concentration may be provided to the blood purification device. Further, since a metering piston pump is not needed to be used, the present invention is disposable and, thus, cleaning and sterilization after blood purification treatment will be unnecessary, thereby resulting in reducing the burdens of healthcare professionals.

### Brief Description of Drawings

Fig. 1 is an explanatory diagram indicating the outline of the configuration of a blood purification system.
Fig. 2 is a front view of a solution generating device.
Fig. 3 is a side view of a solution generating device.
Fig. 4 is a front view of another solution generating device.

### Description of Embodiments

Hereinafter, the preferred embodiments of the present invention will be explained in reference to the drawings. It should be noted that the following embodiments are examples for explaining the present invention and that the present invention will not be limited only to such embodiment. The same reference sign will be applied to the same element and overlapping explanations will be omitted. Further, unless otherwise mentioned, the positional relationship in the drawings, such as top, bottom, left, and right, etc., will be based on the positional relationships indicated in the drawings. Moreover, the dimensional ratios of the drawings are not limited to the illustrated ratios.

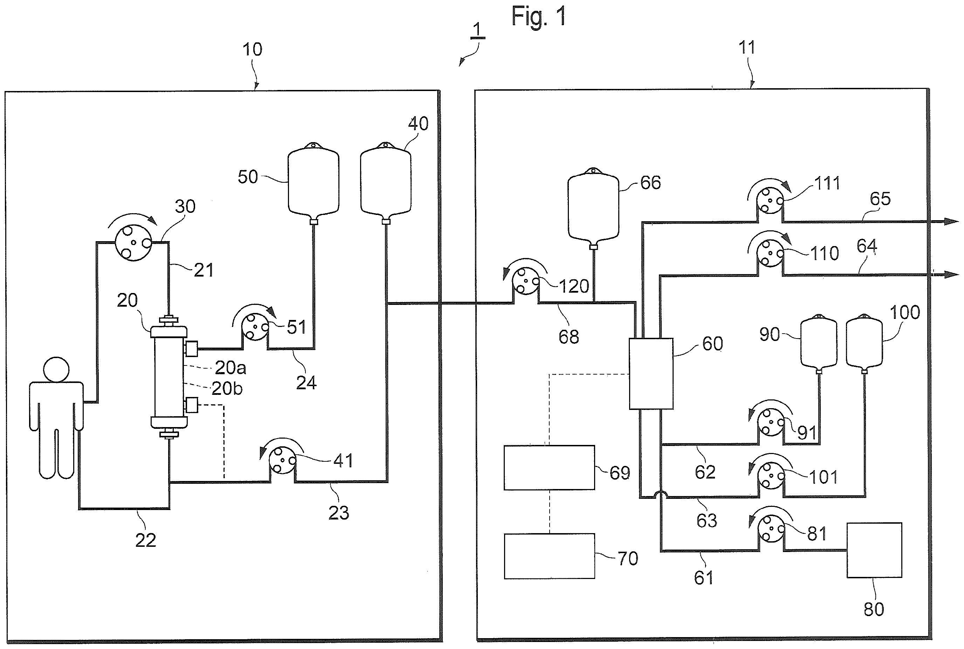

Fig. 1 is an explanatory diagram indicating the outline of the configuration of a blood purification system 1 comprising a solution generating device 60 according to the present embodiment. The blood purification system 1 is, for example, for implementing a continuous blood purification treatment and comprises a blood purification device 10 which performs blood purification treatment on a patient, and a solution supply device 11, which supplies and refills dialysate and fluid replacement solutions to the blood purification device 10.

The blood purification device 10 comprises, for example: a blood purification unit 20; a blood supply circuit 21 for supplying blood of a patient to the blood purification unit 20; a blood return circuit 22 for returning the blood to the patient from the blood purification unit 20; a solution supply circuit 23 for performing at least one of supplying fluid replacement to the blood return circuit 22 or supplying a dialysate to the blood purification unit 20; and a drainage circuit 24 for discharging drainage generated by the blood purification unit 20.

The blood purification unit 20 is, for example, a hollow fiber module including a hollow fiber membrane, wherein the blood purification unit 20 is able to cause the waste product, etc. within the blood supplied to the primary side 20a of the hollow fiber membrane to pass through the secondary side 20b of the hollow fiber membrane so as to filter and purify the blood.

The blood supply circuit 21 is, for example, connected to an inlet of the primary side 20a of the blood purification unit 20 from a needle portion (not illustrated) injected into a patient. A pump 30 is provided in the blood supply circuit 21 and the patient's blood may be supplied to the blood purification unit 20 at a predetermined flow rate. As the pump 30, for example, a so-called tube pump which sends fluid within the tube by pushing the tube configuring the circuit, is used.

The blood return circuit 22 is connected to the needle part (not illustrated) from an outlet of the primary side 20a of the blood purification unit 20.

The solution supply circuit 23 is connected to at least the blood supply circuit 21, the blood return circuit 22, or the secondary side 20b of the blood purification unit 20 from the solution reservoir 40. A selection can be made as to whether the solution supply circuit 23 is connected to the blood supply circuit 21, connected to the blood return circuit 22, connected to the blood purification unit 20, or connected to a plurality thereof according to the type of blood purification treatment which includes, for example, Continuous HemoFiltration (CHF), Continuous HemoDialysis (CHD), and Continuous HemoDiaFiltration (CHDF). A tube pump 41 is provided in the solution supply circuit 23 and the dialysate and fluid replacement solution of the solution reservoir 40 may be supplied at a predetermined flow rate. When connecting to a plurality of elements, for example, the solution supply circuit may be branched (not illustrated) and an additional tube pump may be arranged on the branched circuit.

The drainage circuit 24 is connected to the drainage reservoir 50 from the secondary side 20b of the blood purification unit 20. A tube pump 51 is provided in the drainage circuit 24 and the drainage of the blood purification unit 20 may be discharged into the drainage reservoir 50 at a predetermined fluid rate.

The solution supply circuit 11 comprises, for example: a solution generating device 60; a first circuit 61 for supplying water as a first fluid to the solution generating device 60; a second circuit 62 for supplying undiluted solution A as a second fluid to the solution generating device 60; a third circuit 63 for supplying undiluted solution B as a third fluid to the solution generating device 60; a first gas discharge circuit 64 and a second gas discharge circuit 65 for discharging gas within the solution generating device 60; a solution reservoir 66 which stores the solution generated by the solution generating device 60; a solution supply circuit 67 for supplying the solution generated by the solution generating device 60 to the solution reservoir 66; a solution refill circuit 68 for refilling the solution of the solution reservoir 66 to a blood purification device 10; a concentration meter 69; and a controller 70.

The first circuit 61 is connected to the solution generating device 60 from a water supply source 80. For example, a tube pump 81 may be provided in the first circuit 61 and water from the water supply source 80 may be supplied to the solution generating device 60 at a predetermined flow rate. As the water supplied from the water supply source 80, for example, reverse osmosis filtered water and ultrafiltered water, etc. may be used. It should be noted that a filtration membrane for purifying water may be provided in the water supply source 80.

The second circuit 62 is connected to the solution generating device 60 from the undiluted solution A reservoir 90. A tube pump 91 is provided in the second circuit 62 and the undiluted solution A in the undiluted solution A reservoir 90 may be supplied to the solution generating device 60 at a predetermined flow rate. For example, the first circuit 61 and the second circuit 62 may be converged in front of the solution generating device 60 so as to be connected to the solution generating device 60 by a single flow path. The undiluted solution A is, for example, an undiluted solution of a solution not including sodium bicarbonate.

The third circuit 63 is connected to the solution generating device 60 from the undiluted solution B reservoir 100. The tube pump 101 is provided in the third circuit 63 and the undiluted solution B in the undiluted solution B reservoir 90 may be supplied to the solution generating device 60 at a predetermined flow rate. The undiluted solution B may be, for example, an undiluted solution of a solution including sodium bicarbonate. It should be noted that the undiluted solution A and undiluted solution B may be solutions in which gas will be generated by reacting to each other when mixed together, for example, the undiluted solution A may be sodium chloride, potassium chloride, potassium chloride hydrate, magnesium chloride, sodium acetate, glucose, etc., and the undiluted solution B may be sodium hydrogen carbonate. It should be noted that, in the present embodiment, the solution supply device is configured by the first to third circuits 61-63, pumps 81, 91, 101, water supply source 80, and reservoirs 90, 100. Here, there is no problem in arranging the undiluted solution A reservoir 90 and the undiluted solution B reservoir in reverse, and the arrangement thereof is not intended to be limited.

The first gas discharge circuit 64 is in communication with, for example, the exterior of the solution supply device 11 from the solution generating device 60. A tube pump 110 is provided in the first gas discharge circuit 64.

The second gas discharge circuit 65 is in communication with, for example, the exterior of the solution supply device 11 from the solution generating device 60. A tube pump 111 is provided in the second gas discharge circuit 65.

The solution supply circuit 67 is connected to the solution reservoir 66 from the solution generating device 60. The solution refill circuit 68 is connected to the solution supply circuit 23 of the blood purification device 10 from the solution reservoir 66. A tube pump 120 is provided in the solution refill circuit 68 and the solution of the solution reservoir 66 is able to be supplied to the blood purification device 10 at a predetermined flow rate.

The concentration meter 69 is, for example, a conductivity sensor and it is able to measure the concentration of a mixture of the undiluted solution A, the undiluted solution B, and water mixed in the solution generating device 60.

The controller 70 is a computer for controlling the operation of the entire solution supply device 11 and it controls the operation of the tube pumps 81, 91, 101, 110, 111, and 120 and the on-off valve (not illustrated) so as to control the generation of solution and the refilling of solution. For example, the controller 70 is able to adjust the flow rate of the tube pumps 81, 91, 101 based on the result detected by the below-mentioned sensors 142, 152 of the concentration meter 69 so as to control the concentration of the generated solution.

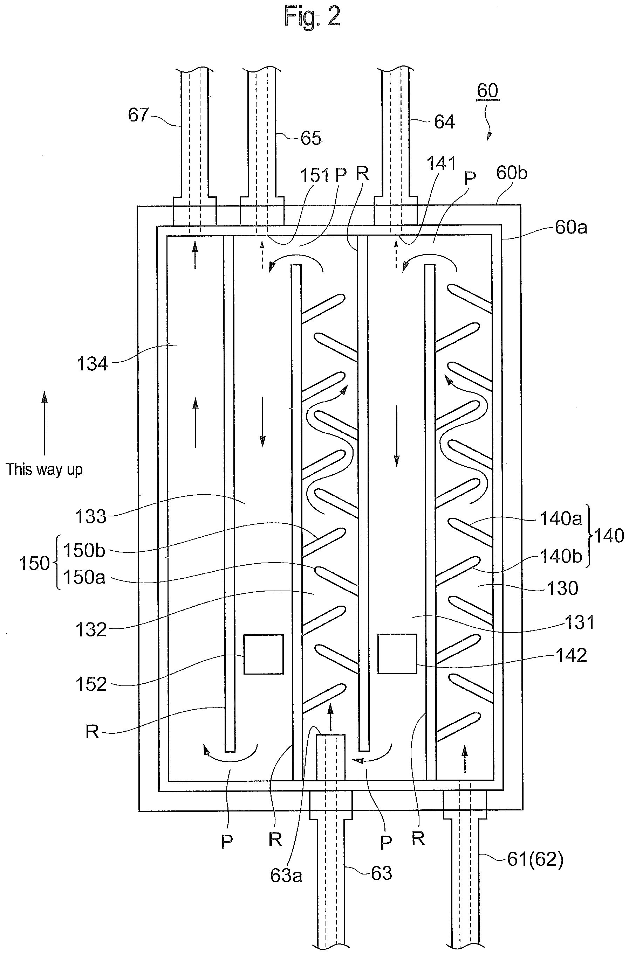

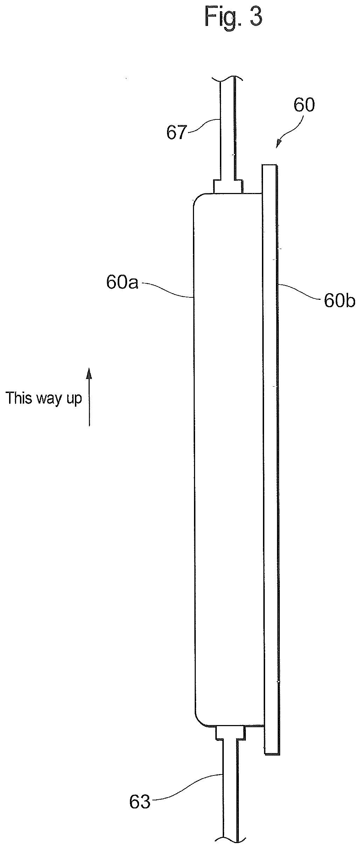

Fig. 2 indicates an example of a front view of a solution generating device 60 and Fig. 3 indicates an example of a side view of a solution generating device 60.

The solution generating device 60 comprises, for example, a quadrate box-shaped casing, and a plurality of lines directed in a vertical direction (flow channels) are provided side-by-side in a horizontal direction within the casing. The solution generating device 60 comprises a line forming section 60a in which a plurality of lines are formed and said lines are exposed, and a transparent plate 60b as a cover plate that closes the exposed surface of the line forming section 60a.

For example, the solution generating device 60 comprises: a first line 130 which causes water and undiluted solution A to flow in an upward direction, thereby mixing them together; a second line 131 which is connected to a top end of the first line 130 and which causes the mixture of water and the undiluted solution A (hereinafter, also referred to as the "first mixture") to flow in a downward direction; a third line 132 connected to a bottom end of the second line 131 and which causes the first mixture and an undiluted solution B to flow in an upward direction, thereby mixing them together; a fourth line 133 connected to a top end of the third line 132 and which causes the mixture of the first mixture and the undiluted solution B (hereinafter, also referred to as the "second mixture") to flow in a downward direction; and a fifth line 134 which is connected to a bottom end of the fourth line 133 and which causes the second mixture to flow in an upward direction.

The lines 130-134 are provided in said order in an approximately parallel line which is in in one direction of a horizontal direction. These lines 130-134 are connected to the adjacent lines at the bottom end or the top end, and are configured so that the flow of fluid will be in the form of a zig-zag flow as a whole. The connections between adjacent lines (lines 130 and 131, lines 131 and 132, lines 132 and 133, lines 133 and 134) are formed by, for example, providing a communication port P in partition R between adjacent lines.

The first circuit 61 (second circuit 62) that supplies water and undiluted solution A is connected to the bottom end (upstream part) of the first line 130. The first line 130 comprises a plurality of baffles 140 arranged along the line 130 as a mixture promoting device which promotes the mixture of water and the undiluted solution A. The baffles 140 include, for example, baffles 140a that protrude from the right wall of a flow path to the left wall and baffles 140b that protrude from the left wall to the right wall, and the baffles 140a and baffles 140b are arranged in an alternating manner from the upstream side to the downstream side. Each of the baffles 140 protrudes obliquely towards the downstream side and reaches beyond the center in a lateral direction of the flow path.

The first line 130 is formed so that, for example, the equivalent diameter within a pipe line (cross-section area of a circuit ÷ total peripheral length of a circuit) of a portion reduced by the baffles 140 in the first line 130 will be 5% or more and 70% or less compared to the equivalent diameter within a pipe line of a portion without the baffles 140 in the same line 130 (including a portion which is assumed to not being including the baffles 140).

In a connection between the upper part of the first line 130 and the second line 131, a gas discharge section 141 is provided that discharges gas collected at such connection. A first gas discharge circuit 64 is connected to the gas discharge section 141. The gas discharge section 141 is provided at the upper portion of the second line 131 side of the connection between the first line 130 and the second line 131.

A sensor 142 for measuring the concentration of the first mixture is provided in the second line 131. The sensor 142 is a sensor for detecting, for example, the conductivity of the first mixture, and the detected conductivity is output to the concentration meter 69. The sensor 142 is arranged below the center in a horizontal direction of the second line 131.

The third circuit 63 which supplies the undiluted solution B is connected to the bottom end (upstream part) of the third line 132. The pipe line of the third circuit 63 extends, for example, within the third line 132 and its supply port 63a is provided on the way up the third line 132 and above the communication port P between the third line 132 and the second line 131.

The third line 132 has a plurality of baffles 150 arranged along the line 132 as a mixture promoting device which promotes the mixture of the first mixture and the undiluted solution B. The baffles 150 have the same configuration as the baffles 140 and include, for example, baffles 150a that protrude from the right wall of a flow path to the left wall and baffles 150b that protrude from the left wall to the right wall, and they are arranged in an alternating manner from the upstream side to the downstream side. Each of the baffles 150 protrudes obliquely towards the downstream side and reaches beyond the center in a lateral direction of the flow path.

The third line 132 is formed so that, for example, the equivalent diameter within a pipe line of a portion reduced by the baffles 150 in the third line 132 will be 5% or more and 70% or less compared to the equivalent diameter within a pipe line of a portion without the baffles 150 in the same line 132.

At a connection between the upper part of the third line 132 and the fourth line 133, a gas discharge section 151 that discharges gas collected at such connection is provided. A second gas discharge circuit 65 is connected to the gas discharge section 151.

A sensor 152 for measuring the concentration of the second mixture is provided in the fourth line 133. The sensor 152 is a sensor for detecting, for example, the conductivity of the first mixture and the detected conductivity is output to the concentration meter 69. The sensor 152 is arranged below the center in a perpendicular direction of the fourth line 133.

The solution supply circuit 67 for supplying the second mixture as the generated solution to the solution reservoir 66 is connected to the top end of the fifth line 134.

It should be noted that, for example, a portion including the solution generating device 60, circuits 61-65, 67, 68, reservoirs 66, 90, 100, and tube pumps 81, 91, 101, 110, 111, 120 is removable from the body of the solution supply device 11 and it may be replaced after each blood purification treatment.

Next, the operation of the solution generating device 60 configured as above will be explained together with the operation of the blood purification system 1 as a whole.

A continuous blood purification treatment for a patient is implemented in the blood purification device 10 indicated in Fig. 1. For example, when a Continuous HemoFiltration (CHF) therapy is implemented, the patient's blood is supplied to the blood purification unit 20 via the blood supply circuit 21 by the tube pump 30. The waste product, etc. removed through a membrane from blood in the blood purification unit 20 is discharged into the drainage reservoir 50 via the drainage circuit 24 by the tube pump 51. Further, by the tube pump 41, the fluid replacement of the solution reservoir 40 is supplied to the blood supply circuit 21 and/or the blood return circuit 22 via the solution supply circuit 23. The blood purified in the blood purification unit 20 is returned to the patient via the blood return circuit 22. Further, when a Continuous HemoDialysis (CHD) therapy is implemented, the dialysate of the solution reservoir 40 is supplied to the secondary side 20b of the membrane of the blood purification unit 20. When a Continuous HemoDiaFiltration (CHDF) therapy is performed, by the tube pump 41, the fluid replacement is supplied to the blood supply circuit 21 and/or the blood return circuit 22 and the dialysate is supplied to the blood purification unit 20.

While a blood purification treatment is being performed in the blood purification device 10, the solution supply device 11 generates a fluid replacement and dialysate solution of a predetermined concentration necessary in the blood purification device 10, and supplies the same to the blood purification device 10. Upon doing so, the tube pumps 81, 91, 101 are operated, water of a predetermined flow rate is supplied from the water supply source 80 to the solution generating device 60 via the first circuit 61, an undiluted solution A of a predetermined flow rate is supplied from the undiluted solution A reservoir 90 to the solution generating device 60 via the second circuit 62, and an undiluted solution B of a predetermined flow rate is supplied from the undiluted solution B reservoir 100 to the solution generating device 60 via the third circuit 63.

In the solution generating device 60 indicated in Fig. 2, water and the undiluted solution A are supplied from the first circuit 61 (second circuit 62) to the first line 130, and as it passes through the first line 130, the mixing is promoted by the baffles 140. The first mixture of water and the undiluted solution A, which were mixed by passing through the first line 130 in an upward direction, turns down at the uppermost part and flows downward in the second line 131. The first mixture which has passed through the second line 131 turns upwards at the lowermost part and flows into the third line 132. The undiluted solution B is supplied from the third circuit 63 to the third line 132 and as it passes through the third line 132, the mixing of the first mixture and the undiluted solution B is promoted by the baffles 150. The second mixture of the first mixture and the undiluted solution B which were mixed by passing through the third line 132 turns downward at the uppermost part and flows downward in the fourth line 133. Then, the second mixture turns upwards at the lowermost part of the fourth line 133 and flows upwards in the fifth line 134 and is discharged into the solution supply circuit 67. The generation speed of the first and second mixtures generated in the solution generating device 60 is set so as to be at a low speed, for example, 300 mL/min or less.

In the solution generating device 60, gas entrapped in the first line 130 and the second line 131 or gas generated therein is collected into the gas discharge section 141 at the uppermost part of the first line 130 and the second line 131. The gas in the gas discharge section 141 is discharged outside via the first gas discharge circuit 64 by the tube pump 110.

Further, gas entrapped in the third line 132 and the fourth line 133, or gas generated therein, is collected into the gas discharge section 151 at the uppermost part of the third line 132 and the fourth line 133. The gas in the gas discharge section 151 is discharged outside via the second gas discharge circuit 65 by the tube pump 111.

In the second line 131, the conductivity of the first mixture is detected by the sensor 142. Further, in the fourth line 133, the conductivity of the second mixture is detected by the sensor 152.

The conductivities detected by the sensors 142, 152 are output to the concentration meter 69, the concentration is measured by the concentration meter 69, and the concentration data thereof is output to the controller 70. The controller 70 adjusts the flow rate of each pump 81, 91, 101 so that the water, the undiluted solution A and the undiluted solution B are mixed at a predetermined ratio, and controls the concentration of the generated solution, based on the concentration data.

The solution generated in the solution generating device 60 is supplied to the solution reservoir 66 via the solution supply circuit 67 and stored therein. The solution of the solution reservoir 66 is supplied to the solution supply circuit 23 of the blood purification device 10 via the solution refill circuit 68 at a predetermined timing by the tube pump 120. The solution supplied to the solution supply circuit 23 is supplied to the solution reservoir 40, the blood return circuit 22 or the blood purification unit 20.

According to the present embodiment, since the baffles 140 are provided in the first line 130 of the solution generating device 60, and mixing of water and the undiluted solution A may be implemented sufficiently in the first line 130. Further, by causing the first mixture to flow upwards in the first line 130, the gas existing inside the first mixture is collected to the connection at the upper part of the first line 130 and the second line 131, and the gas may be effectively removed from the first mixture by the gas discharge section 141 arranged in said connection. In addition, by causing the first mixture to flow downwards in the second line 131, gas existing inside the first mixture is suppressed from passing through to the downstream side, thereby, allowing the concentration of the first mixture in the second line 131 to be correctly measured by the sensor 142 and the flow rate of the tube pumps 81, 91 is able to be adjusted based on said measurement result, thereby strictly controlling the concentration of the first mixture. Hence, the concentration of the solution may be strictly controlled and the solution with a correct concentration may be supplied to the blood purification device 10. Further, since relatively inexpensive tube pumps 81, 91 may be used instead of using the metering piston pump, the solution generating device 60 is disposable. Therefore, there is no need to clean and sterilize the circuit after the blood purification treatment and thus the burden placed on healthcare professionals will be reduced. Moreover, since the gas discharge section 141 is provided at the upper portion of the second line 131 side of the connection between the first line 130 and the second line 131, the gas in the second line 131 where the sensor 142 is present may be effectively discharged and hence the concentration of the first mixture may be measured more accurately by the sensor 142.

Since the sensor 142 is provided in the second line 131, the concentration of the first mixture after the removal of gas may be measured accurately. Hence, the concentration of the solution may be controlled more strictly.

Further, since the gas discharge section 151 is provided in the connection at the upper part of the third line 132 and the fourth line 133, gas may be removed from the second mixture. Therefore, the concentration of the second mixture may be measured with more accuracy by the sensor 152, thereby allowing the concentration of the second mixture to be controlled strictly by adjusting the flow rate of the pump 101. Therefore, the concentration of the finally generated solution may be strictly controlled.

Since the sensor 152 is provided in the fourth line 133, the concentration of the second mixture after the removal of gas may be measured accurately. Hence, the concentration of the solution may be controlled more strictly.

Since the fifth line 134 which causes the second mixture to flow in an upward direction is connected to the fourth line 133, when the reservoir 66 is arranged above the solution generating device 60, the piping may be simplified. It should be noted that the reservoir 66 may be connected from the bottom of the fourth line 133 using a circuit.

The supply port 63a of the pipe line of the third circuit 63 is provided closer to the downstream side than the communication part of the third line 132 and the second line 131, hence preventing the gas mixed in the undiluted solution B and the gas generated by the chemical reaction of the first mixture and the undiluted solution B from entering into the second line 131 side. As a result, the measurement of the concentration may be correctly performed by the sensor 142 without the gas affecting the sensor 142 of the second line 131.

Since a plurality of baffles 140, 150 arranged along the lines are used in the mixture promoting device in the first line 130 and the third line 132, the respective mixing may be performed sufficiently and appropriately.

Moreover, since the equivalent diameter within a pipe line of the portions narrowed by the baffles 140, 150 are 5% or more and 70% or less compared to the equivalent diameter within a pipe line of the portions not having the baffles of the same line, a moderate turbulence is created, thereby enabling mixing to be performed effectively.

Since the body of the solution generating device 60 comprises a quadrate casing and the lines 130-134 are arranged side by side in a horizontal direction in the casing, the device may be compact and inexpensive. Therefore, for example, it will be easy to achieve the disposability of the solution generating device 60.

According to the present embodiment, even if the rate of generation of the generated mixture is 300mL/min or less, which is a low speed, since sufficient mixing may be achieved in the solution generating device 60, and the measurement of concentration by the sensors 142, 152 may be performed correctly, thereby enabling the strict control of the concentration of the solution.

According to the present embodiment, since the solution generated by the solution generating device 60 is supplied in-line to the continuous blood purification device 10 of the blood purification system 1, a solution with a correct concentration may be continuously supplied for a prolonged continuous blood purification treatment. In such a case, although it is necessary to generate a solution at a low flow rate, the mixing of the solution may be performed sufficiently and appropriately using the solution generating device 60 comprising the mixture promoting device. Further, since the mixing may be sufficiently performed, it is possible to accurately measure the concentration of the mixture with the sensor, and as a result, it is possible to accurately adjust the flow rate of the pump and strictly control the concentration of the solution. Further, since the present embodiment is feasible with inexpensive devices without using the metering pump, it may be disposable and as a result cleaning and sterilization after blood purification treatment will be unnecessary, thereby resulting in a reduced burden on healthcare professionals.

Although a preferred embodiment of the present invention has been explained above by referring to the attached drawings, the present invention is not limited to such example. It is obvious that a person skilled in the art will be able to think of various modifications and corrections within the scope of the idea recited in the scope of claims and it is naturally understood that such modifications and corrections belong to the technical scope of the present invention.

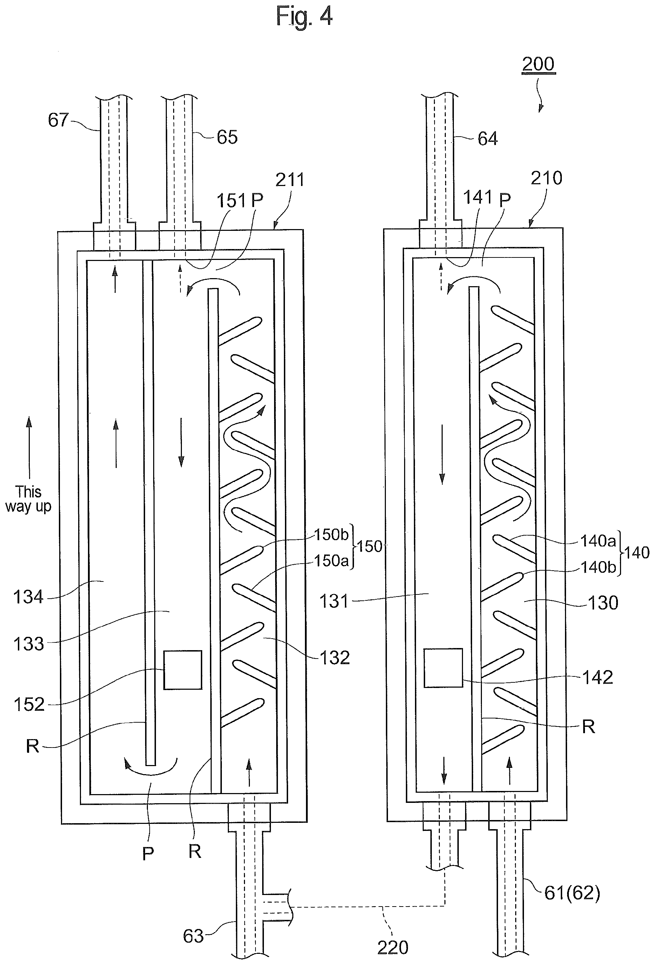

For example, in the above embodiment, although the lines 130-134 were formed in the solution generating device 60, and both the mixing of water and the undiluted solution A and the mixing of such first mixture and the undiluted solution B were performed consecutively, such mixing may also be performed separately. In such a case, as indicated in Fig. 4, for example, the solution generating device 200 can be divided into a first solution generating section 210 which performs mixing of water and the undiluted solution A, and a second solution generating section 211 which performs mixing of the first mixing solution and the undiluted solution B. The first solution generating section 210 may comprise a first line 130 and a second line 131. The second solution generating section 211 comprises a third line 132, fourth line 133, and fifth line 134. The bottom end of the second line 131 may be connected with the bottom end of the third line 132 or a connection circuit 220 leading to a third circuit 63. In such a case, the concentration of the first mixture and the second mixture may be correctly measured and the concentration of the generated solution may be strictly controlled by adjusting the flow rate of the tube pumps 81, 91, 101.

Although a sensor 142 was provided in the second line 131 in the above-mentioned embodiment, the position may not be limited as such and it may be provided at another place, as long as it is downstream from the second line 131 and upstream from the third line 132. For example, as indicated in Fig. 4, if the device is divided into two solution generating sections 210, 211, the sensor 142 may be provided in the connection circuit 220. Further, the sensor 152 may not be limited to the fourth line 133 and it may be at any other place, as long as it is downstream from the third line 132. For example, it may be provided in the fifth line 134 and the solution supply circuit 67.

In the above embodiment, although the fifth line 134 was provided at the downstream side of the fourth line 133, the fifth line 134 is not necessarily needed. In such a case, the solution supply circuit 67 may be directly connected to the bottom end of the fourth line 133.

Although the supply port 63a of the pipe line of the third circuit 63 is formed on the way up the third line 132, it may be connected to the bottom end of the third line 132. Further, although the mixture promoting device of the first line 130 and the third line 132 comprises a plurality of baffles 140, 150 arranged along the lines, it is not limited thereto, and it may be in a form, etc. which generates turbulence by providing walls in the flow channel and arranging holes which communicate with the wall.

Further, although the lines 130-134 within the solution generating device 60 were arranged side by side in a parallel direction, they may be in another arrangement as well. Moreover, although five lines were used in the present embodiment, other numbers of lines may also be adapted in the present invention. Further, the types of fluid to be mixed for generating the solution are not limited to three types and the present invention may be applied to a case in which different numbers of types of solutions (two or more types) are used. Furthermore, the solution generating device 60 of the present invention may be used only for mixing the first fluid (water) and the second fluid (undiluted solution A), or may be used only for mixing the first mixture and the third fluid (undiluted solution B). Further, the shape of the solution generating device 60 may be in another form.

The solution generating device of the present invention may be applied to a case in which a solution is supplied to blood purification devices other than continuous blood purification devices.

### Industrial Applicability

The present invention is useful upon providing a solution generating device and a blood purification system which are disposable, capable of correctly measuring the concentration of the mixture, and capable of strictly controlling the concentration of the generated solution.

### Reference Signs List

1 Blood purification system
10 Blood purification device
11 Solution supply device
60 Solution generating device
130 First line
131 Second line
132 Third line
133 Fourth line
134 Fifth line
140 Baffles
141 First gas discharge section
142 Sensor
150 Baffles
151 Second gas discharge section
152 Sensor

## Claims

1. A dialysate generating device (60) that generates a dialysate used in a blood purification device, wherein the dialysate generating device (60) comprises:
a first line (130) configured to cause a first fluid and a second fluid to flow in an upward direction and to mix the first fluid and the second fluid using a mixture promoting device;
a second line (131) which is connected to a top end of the first line (130) and is configured to cause a first mixture of the first fluid and the second fluid to flow in a downward direction; and
a gas discharge section (141) which is provided at a connection between the first line (130) and the second line (131) and which is configured to discharge gas collected at the connection,
a water supply source (80) configured to supply water as the first fluid, and
a first undiluted solution reservoir (90) configured to supply a first undiluted solution (A) as the second fluid,
**characterized in that**
a sensor configured to measure a concentration of the first mixture is provided in the second line.

2. The dialysate generating device (60) according to claim 1, wherein the gas discharge section (141) is provided at an upper portion of the second line (131) side of the connection.

3. The dialysate generating device (60) according to claim 1 or 2 further comprising:
a third line (132) connected to a bottom end of the second line (131), which is configured to cause the first mixture and a third fluid to flow in an upward direction and to mix the first mixture and the third fluid using a mixture promoting device;
a fourth line (133) connected to a top end of the third line (132), which is configured to cause a second mixture of the first mixture and the third fluid to flow in a downward direction; and
a gas discharge section (151) provided at a connection between the third line (132) and the fourth line (133) and which discharges gas collected at the connection,
a second, undiluted solution reservoir (100) configured to supply a second undiluted solution (B) as the third fluid.

4. The dialysate generating device (60) according to claim 3, wherein the fourth line (133) is provided with a sensor (152) which is configured to measure a concentration of the second mixture.

5. The dialysate generating device (60) according to claim 3 or 4 connected to a bottom end of the fourth line (133) and further comprising a fifth line (134) that is configured to cause the second mixture to flow in an upward direction.

6. The dialysate generating device (60) according to any one of claims 3 to 5, wherein a fluid circuit configured to supply the third fluid is connected to the third line (132), and
a supply port (63a) of the fluid circuit is provided closer to a downstream side than a connection of the second line (131) and the third line (132).

7. The dialysate generating device (60) according to any one of claims 1 to 6, wherein the mixture promoting device is a plurality of baffles (140, 150) arranged along a line (130, 132).

8. The dialysate generating device (60) according to claim 7, wherein an equivalent diameter within a pipe line (cross-section area of a circuit ÷ total peripheral length of a circuit) of a portion reduced by the baffles (140, 150) in a line is 5% or more and 70% or less compared to an equivalent diameter within a pipe line (130, 132) of a portion without the baffles (140, 150) in the same line (130, 140).

9. The dialysate generating device (60) according to any one of claims 1 to 8, wherein a device body comprises a quadrate casing, wherein
at least the first line (130) and the second line (131) are arranged side by side in a horizontal direction within the casing.

10. The dialysate generating device (60) according to any one of claims 1 to 9, wherein a generation speed of a mixture to be generated is 300 mL/min or less.

11. A blood purification system (1) comprising:
a dialysate generating device (60) according to any one of claims 1 to 10;
a dialysate reservoir (66) connected to the dialysate generating device (60) and having a dialysate generated by the dialysate generating device (60) stored therein; and
a continuous blood purification device (10) that is configured to connect to the dialysate reservoir (66) and is supplied with a dialysate of the dialysate reservoir (66).

12. The blood purification system (1) according to claim 11 having a fluid supply device that is configured to supply fluid to the dialysate generating device (60).

## Patentansprüche

1. Dialysat-erzeugende Vorrichtung (60), die ein Dialysat erzeugt, das in einer Blutreinigungsvorrichtung verwendet wird, wobei die Dialysat-erzeugende Vorrichtung (60) umfasst:
eine erste Leitung (130), die so konfiguriert ist, dass sie bewirkt, dass eine erste Flüssigkeit und eine zweite Flüssigkeit nach oben fließen und die erste Flüssigkeit und die zweite Flüssigkeit unter Verwendung einer mischungsfördernden Vorrichtung miteinander gemischt werden;
eine zweite Leitung (131), die an ein oberes Ende der ersten Leitung (130) angeschlossen ist und so konfiguriert ist, dass sie bewirkt, dass ein erstes Gemisch aus der ersten Flüssigkeit und der zweiten Flüssigkeit nach unten fließt; und
einen Gasausleitungsabschnitt (141), der sich an einer Verbindung zwischen der ersten Leitung (130) und der zweiten Leitung (131) befindet und der so konfiguriert ist, dass er an der Verbindung gesammeltes Gas ausleitet;
eine Wasserzufuhrquelle (80), die so konfiguriert ist, dass als erste Flüssigkeit Wasser zugeführt wird; und
ein Reservoir für die erste unverdünnte Lösung (90), das so konfiguriert ist, dass es als zweite Flüssigkeit eine erste unverdünnte Lösung (A) zuführt;
**dadurch gekennzeichnet, dass**
in der zweiten Leitung ein Sensor vorhanden ist, der so konfiguriert ist, dass er eine Konzentration des ersten Gemischs misst.

2. Dialysat-erzeugende Vorrichtung (60) gemäß Anspruch 1, wobei der Gasausleitungsabschnitt (141) an einem oberen Teil der Seite der Verbindung mit der zweiten Leitung (131) vorhanden ist.

3. Dialysat-erzeugende Vorrichtung (60) gemäß Anspruch 1 oder 2, weiterhin umfassend:
eine dritte Leitung (132), die an ein unteres Ende der zweiten Leitung (131) angeschlossen ist und so konfiguriert ist, dass sie bewirkt, dass das erste Gemisch und eine dritte Flüssigkeit nach oben fließen und das erste Gemisch und die dritte Flüssigkeit unter Verwendung einer mischungsfördernden Vorrichtung miteinander gemischt werden;
eine vierte Leitung (133), die an ein oberes Ende der dritten Leitung (132) angeschlossen ist und so konfiguriert ist, dass sie bewirkt, dass ein zweites Gemisch aus dem ersten Gemisch und der dritten Flüssigkeit nach unten fließt; und
einen Gasausleitungsabschnitt (151), der sich an einer Verbindung zwischen der dritten Leitung (132) und der vierten Leitung (133) befindet und der an der Verbindung gesammeltes Gas ausleitet;
ein Reservoir für die zweite unverdünnte Lösung (100), das so konfiguriert ist, dass es als dritte Flüssigkeit eine zweite unverdünnte Lösung (B) zuführt.

4. Dialysat-erzeugende Vorrichtung (60) gemäß Anspruch 3, wobei die vierte Leitung (133) mit einem Sensor (152) versehen ist, der so konfiguriert ist, dass er eine Konzentration des zweiten Gemischs misst.

5. Dialysat-erzeugende Vorrichtung (60) gemäß Anspruch 3 oder 4, die an ein unteres Ende der vierten Leitung (133) angeschlossen ist und weiterhin eine fünfte Leitung (134) umfasst, die so konfiguriert ist, dass sie bewirkt, dass das zweite Gemisch nach oben fließt.

6. Dialysat-erzeugende Vorrichtung (60) gemäß einem der Ansprüche 3 bis 5, wobei ein Flüssigkeitskreislauf, der so konfiguriert ist, dass er die dritte Flüssigkeit zuführt, an die dritte Leitung (132) angeschlossen ist; und
sich ein Zufuhranschluss (63a) des Flüssigkeitskreislaufs näher an einer Stromabwärtsseite als an einer Verbindung der zweiten Leitung (131) und der dritten Leitung (132) befindet.

7. Dialysat-erzeugende Vorrichtung (60) gemäß einem der Ansprüche 1 bis 6, wobei es sich bei der mischungsfördernden Vorrichtung um eine Vielzahl von Strombrechern (140, 150) handelt, die entlang einer Leitung (130, 132) angeordnet sind.

8. Dialysat-erzeugende Vorrichtung (60) gemäß Anspruch 7, wobei ein Äquivalentdurchmesser innerhalb einer Rohrleitung (Querschnittsfläche eines Kreislaufs/Gesamtperipherielänge eines Kreislaufs) eines Teils, der durch die Strombrecher (140, 150) in einer Leitung reduziert ist, 5% oder mehr und 70% oder weniger im Vergleich zu einem Äquivalentdurchmesser innerhalb einer Rohrleitung (130, 132) eines Teils ohne die Strombrecher (140, 150) in derselben Leitung (130, 140) beträgt.

9. Dialysat-erzeugende Vorrichtung (60) gemäß einem der Ansprüche 1 bis 8, wobei ein Korpus der Vorrichtung ein quadratisches Gehäuse umfasst, wobei
wenigstens die erste Leitung (130) und die zweite Leitung (131) innerhalb des Gehäuses in horizontaler Richtung nebeneinander angeordnet sind.

10. Dialysat-erzeugende Vorrichtung (60) gemäß einem der Ansprüche 1 bis 9, wobei die Erzeugungsgeschwindigkeit eines zu erzeugenden Gemischs 300 ml/min oder weniger beträgt.

11. Blutreinigungssystem (1), umfassend:
eine Dialysat-erzeugende Vorrichtung (60) gemäß einem der Ansprüche 1 bis 10;
ein Dialysatreservoir (66), das an die Dialysat-erzeugende Vorrichtung (60) angeschlossen ist und in dem ein Dialysat, das durch die Dialysat-erzeugende Vorrichtung (60) erzeugt wurde, bevorratet wird; und
eine kontinuierliche Blutreinigungsvorrichtung (10), die so konfiguriert ist, dass sie an das Dialysatreservoir (66) angeschlossen ist und mit einem Dialysat des Dialysatreservoirs (66) versorgt wird.

12. Blutreinigungssystem (1) gemäß Anspruch 11, das eine Flüssigkeitszufuhrvorrichtung aufweist, die so konfiguriert ist, dass sie der Dialysaterzeugenden Vorrichtung (60) Flüssigkeit zuführt.

## Revendications

1. Dispositif de production de dialysat (60) qui produit un dialysat utilisé dans un dispositif de purification de sang, dans lequel le dispositif de production de dialysat (60) comprend :
une première ligne (130) configurée pour occasionner qu'un premier fluide et un second fluide s'écoulent dans une direction vers le haut et pour mélanger le premier fluide et le second fluide en utilisant un dispositif de promotion de mélange ;
une seconde ligne (131) qui est connectée à une extrémité de haut de la première ligne (130) et est configurée pour occasionner qu'un premier mélange du premier fluide et du second fluide s'écoule dans une direction vers le bas ; et
une section de décharge de gaz (141) qui est fournie à une connexion entre la première ligne (130) et la seconde ligne (131) et qui est configurée pour décharger du gaz recueilli à la connexion,
une source d'introduction d'eau (80) configurée pour introduire de l'eau comme le premier fluide, et
un réservoir de première solution non diluée (90) configuré pour introduire une première solution non diluée (A) comme le second fluide,
**caractérisé en ce que**
un capteur configuré pour mesurer une concentration du premier mélange est fourni dans la seconde ligne.

2. Dispositif de production de dialysat (60) selon la revendication 1, dans lequel la section de décharge de gaz (141) est fournie sur une portion supérieure du côté de seconde ligne (131) de la connexion.

3. Dispositif de production de dialysat (60) selon la revendication 1 ou 2 comprenant de plus :
une troisième ligne (132) connectée à une extrémité de fond de la seconde ligne (131), qui est configurée pour occasionner que le premier mélange et un troisième fluide s'écoulent dans une direction vers le haut et pour mélanger le premier mélange et le troisième fluide en utilisant un dispositif de promotion de mélange ;
une quatrième ligne (133) connectée à une extrémité de haut de la troisième ligne (132), qui est configurée pour occasionner qu'un second mélange du premier mélange et du troisième fluide s'écoule dans une direction vers le bas ; et
une section de décharge de gaz (151) fournie à une connexion entre la troisième ligne (132) et la quatrième ligne (133) et qui décharge du gaz recueilli à la connexion,
un réservoir de seconde solution non diluée (100) configuré pour introduire une seconde solution non diluée (B) comme le troisième fluide.

4. Dispositif de production de dialysat (60) selon la revendication 3, dans lequel la quatrième ligne (133) est munie d'un capteur (152) qui est configuré pour mesurer une concentration du second mélange.

5. Dispositif de production de dialysat (60) selon la revendication 3 ou 4 connecté à une extrémité de fond de la quatrième ligne (133) et comprenant de plus une cinquième ligne (134) qui est configurée pour occasionner que le second mélange s'écoule dans une direction vers le haut.

6. Dispositif de production de dialysat (60) selon l'une quelconque des revendications 3 à 5, dans lequel un circuit de fluide configuré pour introduire le troisième fluide est connecté à la troisième ligne (132), et
un orifice d'introduction (63a) du circuit de fluide est fourni plus à proximité d'un côté aval qu'une connexion de la seconde ligne (131) et de la troisième ligne (132).

7. Dispositif de production de dialysat (60) selon l'une quelconque des revendications 1 à 6, dans lequel le dispositif de promotion de mélange est constitué de plusieurs chicanes (140, 150) disposées le long d'une ligne (130, 132).

8. Dispositif de production de dialysat (60) selon la revendication 7, dans lequel un diamètre équivalent dans une ligne de tuyau (surface transversale d'un circuit ÷ longueur périphérique totale d'un circuit) d'une portion réduite par les chicanes (140, 150) dans une ligne est de 5 % ou supérieure et de 70 % ou inférieure comparée à un diamètre équivalent dans une ligne de tuyau (130, 132) d'une portion sans les chicanes (140, 150) dans la même ligne (130, 140).

9. Dispositif de production de dialysat (60) selon l'une quelconque des revendications 1 à 8, dans lequel un corps de dispositif comprend un boîtier de cube, dans lequel
au moins la première ligne (130) et la seconde ligne (131) sont disposées côte à côte dans une direction horizontale dans le boîtier.

10. Dispositif de production de dialysat (60) selon l'une quelconque des revendications 1 à 9, dans lequel une vitesse de production d'un mélange à produire est de 300 ml/min ou inférieure.

11. Système de purification de sang (1) comprenant :
un dispositif de production de dialysat (60) selon l'une quelconque des revendications 1 à 10 ;
un réservoir de dialysat (66) connecté au dispositif de production de dialysat (60) et présentant un dialysat produit par le dispositif de production de dialysat (60) stocké dans celui-ci ; et
un dispositif de purification continue de sang (10) qui est configuré pour connecter le réservoir de dialysat (66) et est alimenté avec un dialysat du réservoir de dialysat (66).

12. Système de purification de sang (1) selon la revendication 11 présentant un dispositif d'introduction de fluide qui est configuré pour introduire du fluide dans le dispositif de production de dialysat (60).
